# EUROPEAN PATENT APPLICATION

(11) **EP 4 723 121 A1**
(43) Date of publication of application: **08.04.2026**
(21) Application number: 24814852.0
(22) Date of filing: 29.01.2024
(51) Int. Cl.: G16B 30/00, G06N 20/00, G16B 40/00

(54) **SEQUENCE GENERATION DEVICE AND SEQUENCE GENERATION METHOD**

(30) Priority: 29.05.2023 JP 2023087628
(71) Applicant: Hitachi, Ltd., Tokyo 100-8280 (JP)
(72) Inventor: TOYOMURA Takashi, Tokyo 100-8280 (JP); KIDO Kunihiko, Tokyo 100-8280 (JP); MATSUMORI Masaki, Tokyo 100-8280 (JP)
(74) Representative: MERH-IP Matias Erny Reichl Hoffmann Patentanwälte PartG mbB
(86) International application number: PCT/JP2024/002663
(87) International publication number: WO 2024/247365

(57) **Abstract**

A sequence generation device according to the present disclosure includes a sequence proxy evaluation unit that calculates a proxy evaluation value for a sequence including a plurality of amino acids, and a sequence generation unit that generates the sequence using a learning model, in order to generate various sequences while maintaining high binding affinity. The sequence generation unit changes some of the amino acids in the sequence generated by the learning model with a predetermined probability, selects an amino acid after change using a probability density function such that the amino acid having a characteristic closer to that of the amino acid before change is selected with a higher probability, trains the learning model to generate a sequence indicating a higher proxy evaluation value based on the proxy evaluation value for the sequence after the amino acid is changed, thereby generating a trained model, and generates a generation sequence using the trained model (see FIG. 3).

## Description

### Technical Field

The present disclosure relates to a sequence generation device and a sequence generation method, and particularly relates to generating a sequence including amino acids.

### Background Art

Antibodies are utilized as antibody drugs. An antibody is constituted of 20 kinds of amino acids linked to each other, each consisting of about 1500 amino acids in total. Therefore, in sequences of amino acids constituting one antibody, the number of combinations of amino acids can be about 20 to the power of 1500. It is unrealistic to actually produce such an enormous number of sequences and evaluate them in experiments.

In order to perform the experiments efficiently, it is possible to generate sequences on a computer, evaluate the generated sequences through simulations, and then actually create only promising sequences and evaluate the created sequences through experiments. However, there are many different evaluation items for sequences (binding affinity, viscosity, solubility, immunogenicity, etc.), and only some of the evaluation items can be evaluated through simulations. For this reason, there are many sequences that fail to meet the pass standard because evaluations thereof are low in actual experiments even if they are promising sequences highly evaluated through simulations.

In order to increase the rate of meeting the pass standard in actual experiments, various methods have been proposed.

In PTL 1, mutations are made at previously identified positions of sequences, and sequences having improved affinity scores and stability scores are selected. Mutations are exhaustive, and evaluations based on affinity scores and stability scores are simple rankings.

In PTL 2, amino acid sequences of which threshold amounts with respect to a binding region is the same as that of non-human antibodies and a threshold amount with respect to a heavy-chain and light-chain framework region is the same as that of human antibodies are generated by a generative adversarial network (GAN). There is a penalty for changing a hydrophobic amino acid to another hydrophobic amino acid.

In PTL 3, a model for generating a sequence having high binding affinity using a long short term memory (LSTM) is used. It is characterized by using positions and anteroposterior relationships of amino acids.

In addition, an example using reinforcement learning is also known. For example, sequences are evaluated for binding affinity through simulations, and reinforcement learning is performed to output sequences having higher evaluation values. Here, in general reinforcement learning, only sequences having high binding affinity but similar amino acid configurations may be generated. Sequences with similar configurations also have similar characteristics. Thus, even if a large number of sequences are generated, the sequences may be all annihilated in actual experiments.

To address this problem, in NPL 1, generative flow networks (GFlowNets) are used to generate various sequences having high binding affinity, thereby avoiding annihilation. GFlowNets are characterized in that a Q function is trained such that the sum of probability values until reaching a certain state in exploration for reinforcement learning matches the sum of probability values after the state. General reinforcement learning, which uses a monotonous Q function corresponding to a fixed discount rate, provided guidance to a single path with a high final reward, but GFlowNets, which provides a more appropriate action value for each state, enables sampling from a variety of modes. The Q function trained in this manner calculates a Q value for each of the 20 candidates for the next amino acid based on the partial sequence generated so far. The amino acid candidate having the highest Q value is selected as the next amino acid and incorporated into the generated sequence.

Here, if amino acids are always selected based on their Q values, it is difficult to select an amino acid having a low Q function at that point in time. This may result in overlooking the possibility of constructing better sequences using unselected amino acids. In order to solve this problem, GFlowNets have a function (mutation function) of randomly changing amino acids with a certain probability.

### Citation List

### Patent Literature

PTL 1: US 2020/0126640 A1
PTL 2: US 2023/0005567 A1
PTL 3: US 2022/0253669 A1

### Non Patent Literature

NPL 1: Jain, M. et al., "Biological Sequence Design with GFlowNets", arXiv: 2203.04115, DOI: 10.48550/arXiv.2203.04115, [online], 2022, [Searched on April 11, 2023], Internet <URL: https://arxiv.org/abs/2203.04115>

### Summary of Invention

### Technical Problem

However, the conventional technologies have a problem that it is difficult to generate various sequences while maintaining high binding affinity.

For example, when amino acids are randomly changed as in NPL 1, the characteristics of the antibody may be greatly changed even if only one amino acid is changed. In such a case, sequences having low binding affinity are generated, resulting in a low meeting rate in actual experiments. Therefore, it is necessary to change amino acids appropriately and not too drastically.

In view of such a situation, the present disclosure provides a sequence generation device and a sequence generation method capable of generating various sequences while maintaining high binding affinity.

### Solution to Problem

As an example of a sequence generation device according to the present disclosure, the sequence generation device includes:
a sequence proxy evaluation unit which calculates a proxy evaluation value for a sequence including a plurality of amino acids; and
a sequence generation unit which generates the sequence using a learning model,
in which the sequence generation unit is configured to:
   - change some of the amino acids in the sequence generated by the learning model with a predetermined probability, and select an amino acid after change using a probability density function such that the amino acid having a characteristic closer to that of the amino acid before change is selected with a higher probability;
   - train the learning model to generate a sequence indicating a higher proxy evaluation value based on the proxy evaluation value for the sequence after the amino acid is changed, thereby generating a trained model; and
   - generate a generation sequence using the trained model.

As an example of a sequence generation method according to the present disclosure, the sequence generation method includes:
a sequence proxy evaluation step of calculating a proxy evaluation value for a sequence including a plurality of amino acids; and
a sequence generation step of generating the sequence using a learning model,
in which the sequence generation step includes:
   - changing some of the amino acids in the sequence generated by the learning model with a predetermined probability, and selecting an amino acid after change using a probability density function such that the amino acid having a characteristic closer to that of the amino acid before change is selected with a higher probability;
   - training the learning model to generate a sequence indicating a higher proxy evaluation value based on the proxy evaluation value for the sequence after the amino acid is changed, thereby generating a trained model; and
   - generating a generation sequence using the trained model.

### Advantageous Effects of Invention

The sequence generation device and the sequence generation method according to the present disclosure can generate various sequences while maintaining high binding affinity.

### Brief Description of Drawings

[FIG. 1] FIG. 1 illustrates an example of a configuration of a sequence generation device according to a first embodiment of the present disclosure.
[FIG. 2] FIG. 2 is a diagram illustrating an outline of processing in the sequence generation device of FIG. 1.
[FIG. 3] FIG. 3 is a flowchart illustrating a flow of processing of the sequence generation device of FIG. 1.
[FIG. 4] FIG. 4 illustrates an example of a characteristic serving as a selection standard.
[FIG. 5] FIG. 5 illustrates a specific example of a probability density function.
[FIG. 6] FIG. 6 illustrates an example of a configuration of a sequence generation device according to a second embodiment.
[FIG. 7] FIG. 7 is a diagram illustrating an outline of processing in the sequence generation device of FIG. 6.
[FIG. 8] FIG. 8 is a flowchart illustrating a flow of processing of the sequence generation device of FIG. 6.
[FIG. 9] FIG. 9 illustrates an example of a configuration of a sequence generation device according to a third embodiment.
[FIG. 10] FIG. 10 is a flowchart illustrating a flow of processing of the sequence generation device in FIG. 10.
[FIG. 11] FIG. 11 illustrates an example of a configuration of a sequence generation device according to a fourth embodiment.
[FIG. 12] FIG. 12 is a flowchart illustrating a flow of processing of the sequence generation device of FIG. 11.

### Description of Embodiments

Hereinafter, embodiments of the present disclosure will be described with reference to the accompanying drawings.

### [First Embodiment]

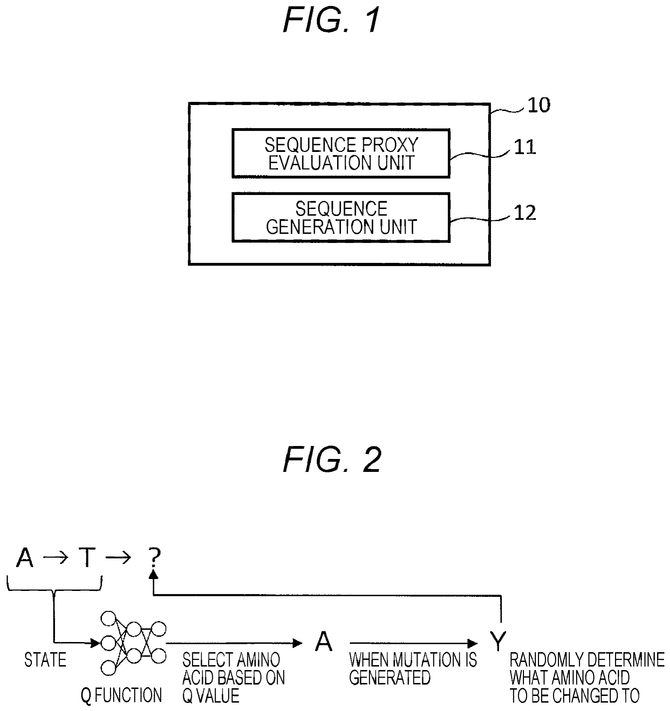

FIG. 1 illustrates an example of a configuration of a sequence generation device 10 according to the first embodiment of the present disclosure. The sequence generation device 10 is a device that generates a sequence including a plurality of amino acids. The sequence is, for example, a sequence of amino acids in an antibody.

The sequence generation device 10 includes a sequence proxy evaluation unit 11 and a sequence generation unit 12. The sequence proxy evaluation unit 11 calculates a proxy evaluation value for a sequence using a function such as multi layer perceptron (MLP). The sequence generation unit 12 generates a sequence using a learning model.

The sequence generation device 10 has a hardware configuration as a known computer, and for example, includes a calculation means and a storage means. The calculation means includes, for example, a processor, and the storage means includes, for example, storage media such as a semiconductor memory device and a magnetic disk device. Some or all of the storage media may be non-transitory storage media.

In addition, the sequence generation device 10 may include input/output means. The input/output means includes, for example, input devices such as a keyboard and a mouse, output devices such as a display and a printer, and communication devices such as a network interface.

The storage means of the sequence generation device 10 may store a program. When the processor executes this program, the computer may implement the functions to be described in the present embodiment, thereby constituting the sequence generation device 10.

An outline of processing in the sequence generation device 10 will be described with reference to FIG. 2. The Q function is a learning model that can be generated by machine learning, and one described in NPL 1 can be used. The input of the Q function is a state representing a partial sequence (a sequence consisting of 0 or more amino acids) generated so far, and includes two amino acids in the example of FIG. 2. The state is empty (0 amino acids) at the start of generation, and becomes longer as the generation processing proceeds.

In FIG. 2, each of the different types of amino acids (20 types in total) is represented by one different alphabet letter according to the practice, and the same applies to the other drawings and the following description.

The output of the Q function is a Q value for each of the 20 types of amino acids. The sequence generation unit 12 selects an amino acid having the highest Q value according to the Q function at that time.

After the amino acid is selected, whether to generate a mutation is determined. When a mutation is to be generated, an amino acid after change is randomly selected using a probability density function.

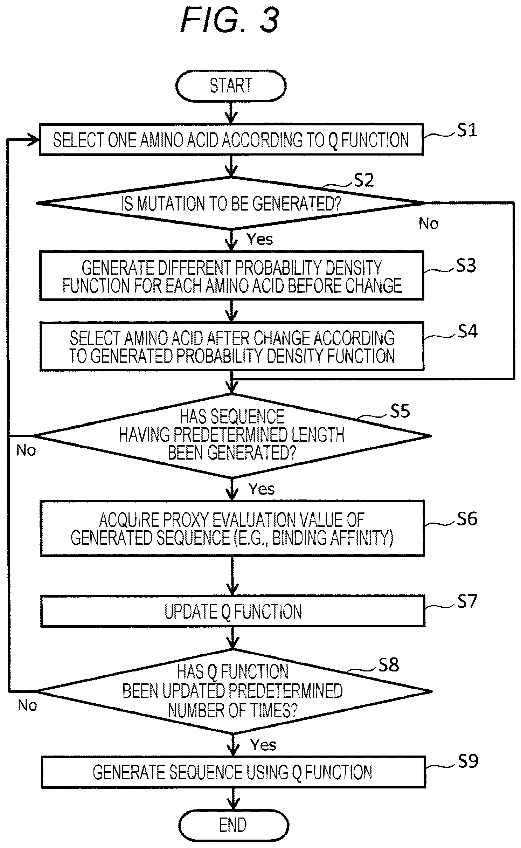

FIG. 3 is a flowchart illustrating a flow of processing of the sequence generation device 10. The sequence generation device 10 can generate a sequence by executing the method according to this flowchart. Hereinafter, the operation of the sequence generation device 10 will be described with reference to the flowchart.

In the processing of FIG. 3, steps S1 to S8 are Q function learning processing, and a Q function is generated as a model trained thereby. First, the sequence generation unit 12 selects amino acids one by one in order from the beginning of the sequence according to the Q function (step S1).

Next, the sequence generation unit 12 determines whether to generate a mutation based on a certain probability determined in advance (step S2).

When it is determined that a mutation is to be generated, the sequence generation unit 12 generates a probability density function for selecting an amino acid after change (step S3). Here, the probability density function varies depending on the amino acid before change, and the probability density function is generated such that an amino acid having a characteristic closer to that of the amino acid before change is selected with a higher probability.

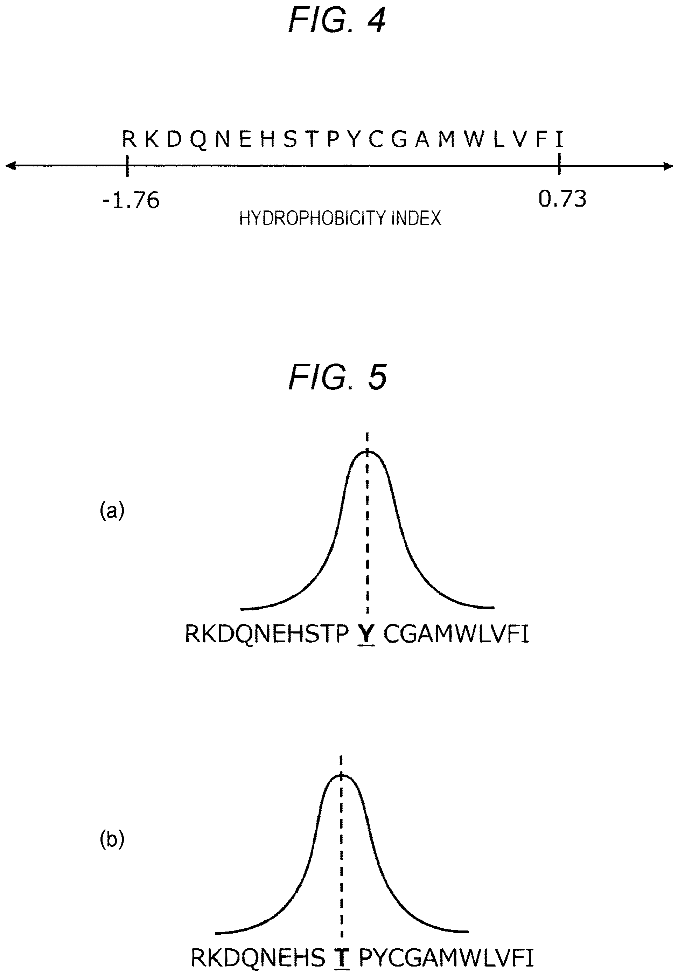

FIG. 4 illustrates an example of a characteristic serving as a selection standard. In this example, the characteristic includes hydrophobicity. As a value representing hydrophobicity, Eisenberg's hydrophobicity index can be used. By using the hydrophobicity index, the closeness of the characteristic of the amino acid can be expressed numerically. The hydrophobicity is one of the important indicators that affect various evaluation items (solubility, aggregation, binding affinity, etc.) of antibody drugs, and by selecting an amino acid based on the hydrophobicity, it is possible to maintain a higher rate of meeting the pass standard in experiments.

FIG. 5 illustrates a specific example of a probability density function. The probability density function can use, for example, a discrete Gaussian distribution, and the horizontal axis can represent a hydrophobicity index. The probability density function in FIG. 5 is a function in which the probability gradually decreases as the difference between the hydrophobicity index of the amino acid before change and the hydrophobicity index of the amino acid after change increases.

FIG. 5(a) illustrates a probability density function in a case where the amino acid before change is Y, in which an amino acid having a characteristic closer to that of Y is selected with a higher probability. FIG. 5(b) illustrates a probability density function in a case where the amino acid before change is T, in which an amino acid having a characteristic closer to that of T is selected with a higher probability.

Here, the probability density function is designed to allow mutations from all amino acids to all amino acids, thus increasing sequence diversity. That is, regardless of the amino acid before change, any amino acid is selected with a different probability. When the same amino acid as the amino acid before change is selected, the sequence becomes the same as that in the case where no mutation is generated.

The sequence generation unit 12 selects an amino acid after change using the probability density function generated in step S3 (step S4).

When it is determined in step S2 not to generate a mutation, steps S3 and S4 are not executed, that is, the amino acid selected in step S1 is used as it is.

The sequence generation unit 12 adds the amino acid selected in step S1 or S4 to the end of the sequence, and determines whether a sequence having a predetermined length has been generated (step S5). If not, the processing returns to step S1, that is, a next amino acid is added.

When a sequence having a predetermined length has been generated, the sequence proxy evaluation unit 11 acquires a proxy evaluation value of the generated sequence (step S6). A method for obtaining a specific evaluation value based on the amino acid sequence can be appropriately designed by those skilled in the art based on known technologies and the like.

A specific example of the proxy evaluation value can be expressed as a value representing binding affinity between an antibody indicated by the sequence and an antigen. By using such a proxy evaluation value, it is possible to give a high proxy evaluation value to a sequence having a high rate of meeting the pass standard for the binding affinity in an experiment.

Note that, in a case where one or more mutations have been generated (which is general with a high probability), this proxy evaluation value can be said to be a proxy evaluation value for the sequence after the amino acid has been changed.

Next, the sequence generation unit 12 acquires the proxy evaluation value from the sequence proxy evaluation unit 11, and trains and updates the Q function to generate a sequence indicating a higher proxy evaluation value based on the acquired proxy evaluation value (step S7).

Next, the sequence generation unit 12 determines whether the Q function has been updated a predetermined number of times (step S8). When the predetermined number of times has not been reached, the processing returns to step S1, that is, a next sequence is generated.

When the predetermined number of times has been reached, the machine learning of the Q function is completed, and the Q function as a trained model is generated. The sequence generation unit 12 generates a sequence (generation sequence) by using the generated Q function (step S9).

As described above, in the first embodiment, in the Q function training processing, the sequence generation unit 12 changes some amino acids in the sequence generated by the Q function with a predetermined probability. Here, the probability density function is used such that an amino acid having a characteristic (e.g., hydrophobicity) closer to that of the amino acid before change is selected with a higher probability. Therefore, a sequence having a closer characteristic (e.g., a sequence having high binding affinity) can be generated, and the rate of meeting the pass standard in tactual experiments is improved.

### [Second Embodiment]

In the second embodiment, a plurality of sequence proxy evaluation units that calculate different proxy evaluation values are used instead of the single sequence proxy evaluation unit in the first embodiment. Hereinafter, the second embodiment will be described, but the description of parts common to the first embodiment may be omitted.

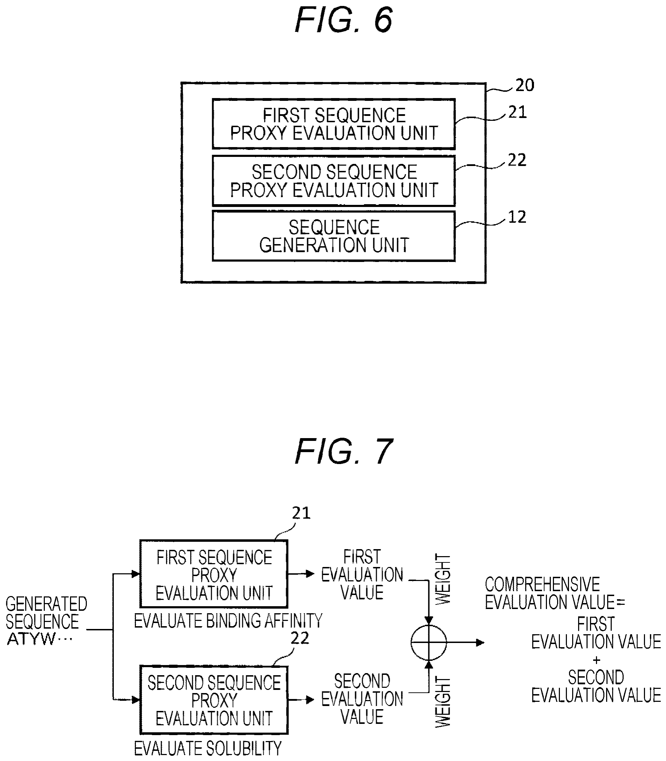

FIG. 6 illustrates an example of a configuration of a sequence generation device 20 according to the second embodiment. The sequence generation device 20 includes, as sequence proxy evaluation units, a first sequence proxy evaluation unit 21 that calculates a first proxy evaluation value indicating a first characteristic of the sequence, and a second sequence proxy evaluation unit 22 that calculates a second proxy evaluation value indicating a second characteristic of the sequence. In addition, the sequence generation device 20 includes a sequence generation unit 12 similar to that of the first embodiment (FIG. 1).

An outline of processing in the sequence generation device 20 will be described with reference to FIG. 7. Various methods may be considered to calculate a proxy evaluation value of a sequence, and a plurality of methods can be combined. A plurality of proxy evaluation values are calculated for the sequence, and a result (added proxy evaluation value) of adding the plurality of proxy evaluation values is set as a comprehensive proxy evaluation value.

In the example of FIG. 7, the first characteristic represents binding affinity between an antibody indicated by the sequence and an antigen, and the second characteristic represents solubility of the antibody indicated by the sequence. The definition of solubility can be appropriately determined by those skilled in the art, and it refers to, for example, solubility in water, physiological saline, or any body fluid. Using such characteristics increases the possibility of meeting the pass standard in experiments.

The sequence generation unit 12 calculates a comprehensive proxy evaluation value based on the first proxy evaluation value and the second proxy evaluation value. For example, the first proxy evaluation value and the second proxy evaluation value may be simply added, but a more flexible evaluation is possible if the comprehensive proxy evaluation value is calculated based on weighted addition.

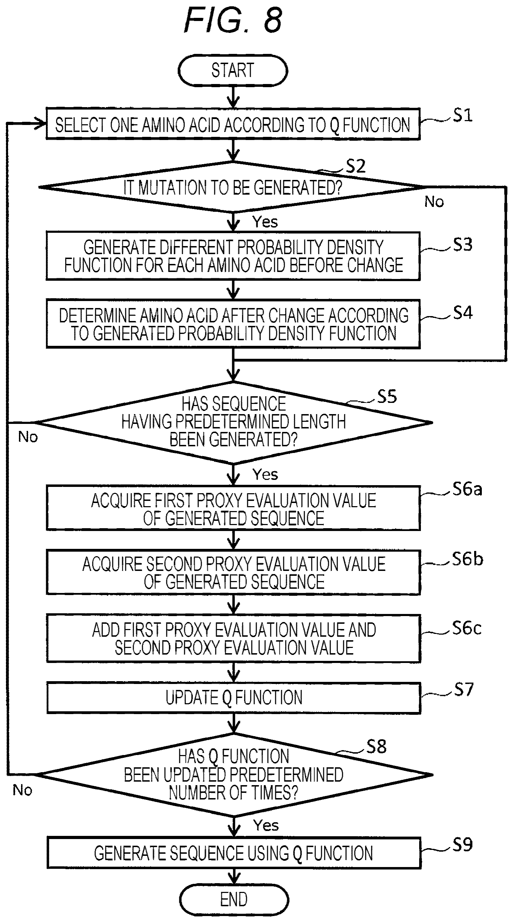

FIG. 8 is a flowchart illustrating a flow of processing of the sequence generation device 20. Step S6 in the first embodiment (FIG. 3) is replaced with steps S6a to S6c in the second embodiment (FIG. 8). The other steps may be similar to those in the first embodiment. Hereinafter, only steps S6a to S6c will be described.

After a sequence having a predetermined length has been generated, the first sequence proxy evaluation unit 21 acquires a first proxy evaluation value of the generated sequence (step S6a), and the second sequence proxy evaluation unit 22 acquires a second proxy evaluation value of the generated sequence (step S6b). As described above, an example of the first characteristic related to the first proxy evaluation value is binding affinity, and an example of the second characteristic related to the second proxy evaluation value is solubility.

Next, the sequence generation unit 12 adds the first proxy evaluation value and the second proxy evaluation value (for example, by weighted addition) to calculate a comprehensive proxy evaluation value (step S6c).

As described above, in the second embodiment, it is possible to generate a sequence incorporating evaluations of actual experiments for a plurality of characteristics, thereby further improving the rate of meeting the pass standard in actual experiments.

Although two types of characteristics are used in the example of FIG. 7, three or more types of characteristics can be used by providing three or more sequence proxy evaluation units.

As a modification of the second embodiment, the probability density function may be changed based on other conditions. For example, when the solubility evaluation value of the sequence generated so far is small (for example, when the solubility evaluation value in water or a predetermined body fluid is equal to or smaller than a predetermined threshold), the right half of the probability density function may be replaced with the probability value of zero. That is, an amino acid having higher hydrophobicity than the amino acid before change may not be selected.

### [Third Embodiment]

The third embodiment is a modification of the first embodiment, in which an evaluation of an actual experiment is fed back to a simulation. Hereinafter, the third embodiment will be described, but the description of parts common to the first embodiment may be omitted.

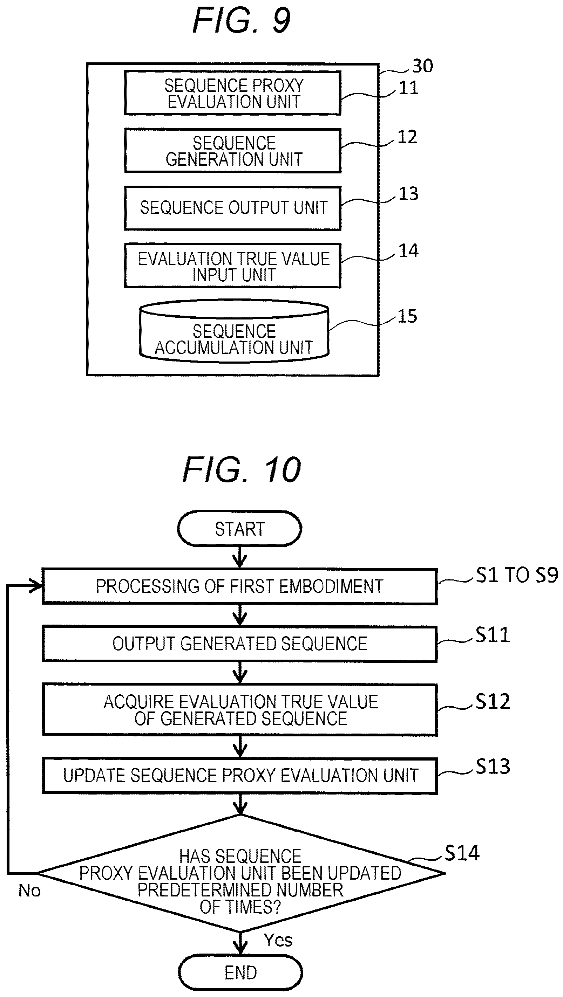

FIG. 9 illustrates an example of a configuration of a sequence generation device 30 according to the third embodiment. The sequence generation device 30 includes a sequence proxy evaluation unit 11 and a sequence generation unit 12 similar to those in the first embodiment (FIG. 1). The sequence generation device 30 further includes a sequence output unit 13 including an output device, an evaluation true value input unit 14 including an input device, and a sequence accumulation unit 15 including a storage device.

FIG. 10 is a flowchart illustrating a flow of processing of the sequence generation device 30. First, the sequence generation device 30 generates a sequence by the processing of the first embodiment or by processing similar to the processing of the first embodiment.

Next, the sequence output unit 13 outputs the generated sequence (step S11). The output is performed to the outside of the sequence generation device 30 by, for example, display using a display device, printing using a printing device, transmission via a communication network, or the like.

Although not illustrated in FIG. 10, after step S11, the user of the sequence generation device 30 evaluates the output sequence through an experiment, acquires an actual evaluation value (evaluation true value) for the sequence, and inputs the actual evaluation value to the sequence generation device 30. The input is performed from the outside of the sequence generation device 30, for example, using a keyboard or a touch panel or by transmission via a communication network, or the like.

Accordingly, the evaluation true value input unit 14 acquires the evaluation true value for the sequence output in step S11 (step S12). Thereafter, the sequence accumulation unit 15 stores a set of the sequence output in step S11 and the evaluation true value acquired in step S12.

Next, the sequence proxy evaluation unit 11 acquires the set of the sequence and the evaluation true value from the sequence accumulation unit 15, and updates itself by performing machine learning based on the acquired set (step S13). For example, the function giving the proxy evaluation value is updated, and accordingly, the sequence proxy evaluation unit 11 is updated.

Next, the sequence proxy evaluation unit 11 determines whether the sequence proxy evaluation unit 11 has been updated a predetermined number of times (step S14). When the predetermined number of times has not been reached, the processing returns to steps S1 to S9, that is, a next sequence is generated. When the predetermined number of times has been reached, the processing of FIG. 10 ends.

As described above, in the third embodiment, the accuracy of the proxy evaluation value is improved using the evaluation true value based on the actual experiment result, thereby making it possible to reduce a situation in which the generated sequence meets the pass standard in the simulation but fail to meet the pass standard in the actual experiment.

In the third embodiment, the same modifications as those in the first and second embodiments can also be made.

### [Fourth Embodiment]

The fourth embodiment is a modification of the second embodiment in combination with the third embodiment, in which the weighting can be optimized at the time of adding two types of characteristics. In particular, a plurality of sequence generation units having different weights are prepared, and an algorithm (so-called multi-arm bandit algorithm) in for adopting a sequence generation unit having the largest average value of weighted evaluation values is used. Hereinafter, the fourth embodiment will be described, but the description of parts common to any of the first to third embodiments may be omitted.

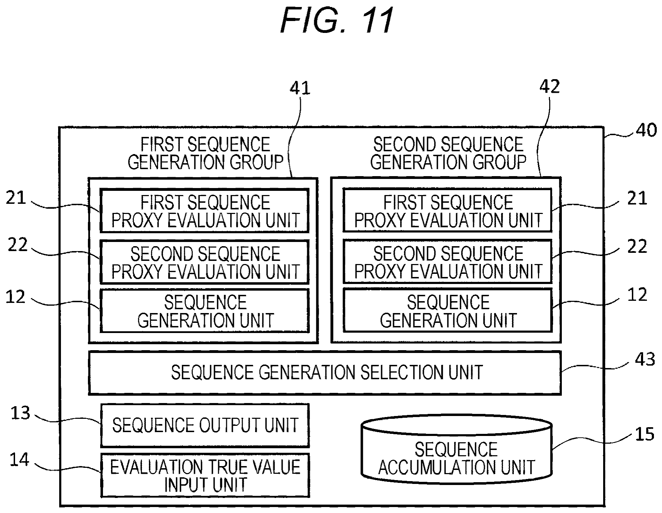

FIG. 11 illustrates an example of a configuration of a sequence generation device 40 according to the fourth embodiment. The sequence generation device 40 includes a first sequence generation group 41, a second sequence generation group 42, and a sequence generation selection unit 43. Each of the first sequence generation group 41 and the second sequence generation group 42 includes a first sequence proxy evaluation unit 21, a second sequence proxy evaluation unit 22, and a sequence generation unit 12 similar to those in the second embodiment (FIG. 6).

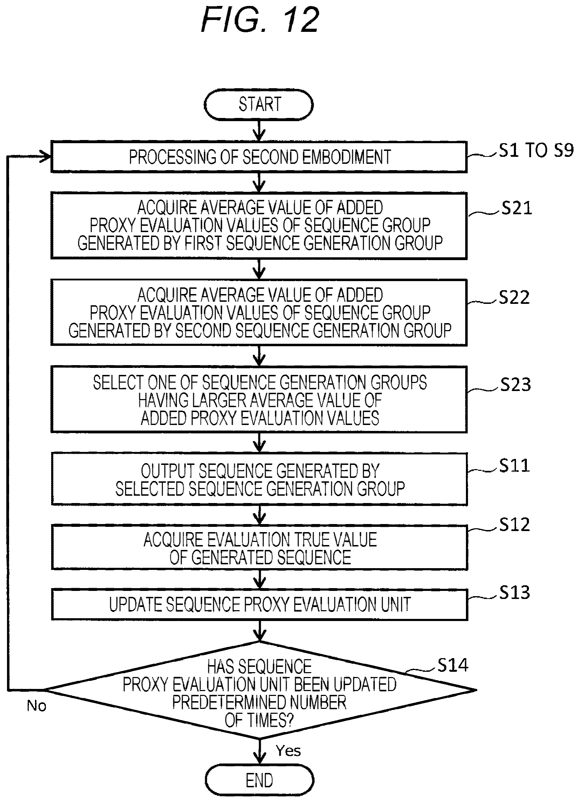

FIG. 12 is a flowchart illustrating a flow of processing of the sequence generation device 40. First, the sequence generation device 40 generates a plurality of sequences by the processing of the second embodiment or by processing similar to the processing of the second embodiment.

Next, the first sequence generation group 41 calculates proxy evaluation values (added proxy evaluation values) based on weighted addition of the first proxy evaluation value and the second proxy evaluation values for each of the generated sequences, and then calculates an average value of the added proxy evaluation values (step S21). The average value is an example of a method of calculating a representative value from a plurality of added proxy evaluation values, and a median value or a maximum value of the added proxy evaluation values may be used. Here, the weighted addition is performed according to a first ratio. As a specific example, a value obtained by multiplying the first proxy evaluation value by 0.7 and a value obtained by multiplying the second proxy evaluation value by 0.3 are added.

Next, the second sequence generation group 42 similarly calculates proxy evaluation values (added proxy evaluation values) based on the weighted addition of the first proxy evaluation value and the second proxy evaluation value for each of the generated sequences, and then calculates an average value of the added proxy evaluation values (step S22). Here, the weighted addition is performed according to a second ratio different from the first ratio used by the first sequence generation group 41. As a specific example, a value obtained by multiplying the first proxy evaluation value by 0.3 and a value obtained by multiplying the second proxy evaluation value by 0.7 are added.

Next, the sequence generation selection unit 43 selects one of the first sequence generation group 41 and the second sequence generation group 42 based on the average value of the added proxy evaluation values calculated by the first sequence generation group 41 and the average value of the added proxy evaluation values calculated by the second sequence generation group 42 (step S23). For example, one having the larger average value is selected.

Next, the sequence output unit 13 outputs a sequence generated by the sequence generation group selected by the sequence generation selection unit 43 (step S11).

Next, the evaluation true value input unit 14 acquires an evaluation true value for the sequence output in step S11 (step S12). Here, in the fourth embodiment, a plurality of characteristics are used, and accordingly, a plurality of evaluation true values are acquired. For example, an evaluation true value for binding affinity and an evaluation true value for solubility are acquired.

Next, each of the first sequence generation group 41 and the second sequence generation group 42 acquires a set of a sequence and an evaluation true value, and performs machine learning based on the acquired set to update itself (step S13). For example, each of the first sequence proxy evaluation units 21 is updated based on the evaluation true value for binding affinity, and each of the second sequence proxy evaluation units 22 is updated based on the evaluation true value for solubility.

As described above, in the fourth embodiment, the weighting can be optimized at the time of adding two types of characteristics, and learning is performed based on a sequence for which a better addition evaluation value has been obtained, thereby further improving the rate of meeting the pass standard in an actual experiment for the generated sequence.

In the example of FIGS. 11 and 12, two sequence generation groups are used, but three or more sequence generation groups can be used. In this case, in step S23, one sequence generation group having the largest average value is selected from among the three or more sequence generation groups.

In the fourth embodiment, the same modifications as those in the first to third embodiments can also be made.

Those skilled in the art can arbitrarily add, change, or delete the components within the scope of the present disclosure in the above-described the first to fourth embodiments and the modifications thereof. For example, the sequence generation device can be constituted by a plurality of computers connected via a communication network.

### Reference Signs List

10, 20, 30, 40 sequence generation device
11 sequence proxy evaluation unit
12 sequence generation unit
13 sequence output unit
14 evaluation true value input unit
15 sequence accumulation unit
21 first sequence proxy evaluation unit
22 second sequence proxy evaluation unit
41 first sequence generation group
42 second sequence generation group
43 sequence generation selection unit

## Claims

1. A sequence generation device comprising:
a sequence proxy evaluation unit which calculates a proxy evaluation value for a sequence including a plurality of amino acids; and
a sequence generation unit which generates the sequence using a learning model,
wherein the sequence generation unit is configured to:
- change some of the amino acids in the sequence generated by the learning model with a predetermined probability, and select an amino acid after change using a probability density function such that the amino acid having a characteristic closer to that of the amino acid before change is selected with a higher probability;
- train the learning model to generate a sequence indicating a higher proxy evaluation value based on the proxy evaluation value for the sequence after the amino acid is changed, thereby generating a trained model; and
- generate a generation sequence using the trained model.

2. The sequence generation device according to claim 1, wherein the proxy evaluation value represents binding affinity between an antibody indicated by the sequence and an antigen.

3. The sequence generation device according to claim 1, wherein the characteristic includes hydrophobicity.

4. The sequence generation device according to claim 1, wherein the probability density function is a function in which the probability gradually decreases as a difference between a hydrophobicity index of the amino acid before change and a hydrophobicity index of the amino acid after change increases.

5. The sequence generation device according to claim 1, wherein
the sequence proxy evaluation unit includes:
a first sequence proxy evaluation unit which calculates a first proxy evaluation value indicating a first characteristic of the sequence; and
a second sequence proxy evaluation unit which calculates a second proxy evaluation value indicating a second characteristic of the sequence, and
the sequence proxy evaluation unit calculates the proxy evaluation value based on the first proxy evaluation value and the second proxy evaluation value.

6. The sequence generation device according to claim 5, wherein
the first characteristic represents binding affinity between an antibody indicated by the sequence and an antigen, and
the second characteristic represents solubility of the antibody indicated by the sequence.

7. The sequence generation device according to claim 5, wherein the sequence proxy evaluation unit calculates the proxy evaluation value based on weighted addition of the first proxy evaluation value and the second proxy evaluation value.

8. The sequence generation device according to claim 1, further comprising a sequence output unit, an evaluation true value input unit, and a sequence accumulation unit,
wherein the sequence output unit outputs the generation sequence,
the evaluation true value input unit acquires an evaluation true value for the generation sequence,
the sequence accumulation unit stores a set of the generation sequence and the evaluation true value, and
the sequence proxy evaluation unit acquires the set of the generation sequence and the evaluation true value from the sequence accumulation unit, and performs machine learning based on the acquired set.

9. The sequence generation device according to claim 8, further comprising a first sequence generation group, a second sequence generation group, and a sequence generation selection unit,
wherein each of the first sequence generation group and the second sequence generation group includes:
- a first sequence proxy evaluation unit which calculates a first proxy evaluation value indicating a first characteristic of the sequence; and
- a second sequence proxy evaluation unit which calculates a second proxy evaluation value indicating a second characteristic of the sequence,
the first sequence generation group calculates the proxy evaluation value by weighted addition of the first proxy evaluation value and the second proxy evaluation value according to a first ratio,
the second sequence generation group calculates the proxy evaluation value by weighted addition of the first proxy evaluation value and the second proxy evaluation value according to a second ratio different from the first ratio,
the sequence generation selection unit selects one from among sequence generation groups including the first sequence generation group and the second sequence generation group based on an average value of the plurality of proxy evaluation values calculated by the first sequence generation group and an average value of the plurality of proxy evaluation values calculated by the second sequence generation group, and
the sequence output unit outputs the generation sequence generated by the one sequence generation group selected by the sequence generation selection unit.

10. A sequence generation method comprising:
a sequence proxy evaluation step of calculating a proxy evaluation value for a sequence including a plurality of amino acids; and
a sequence generation step of generating the sequence using a learning model,
wherein the sequence generation step includes:
- changing some of the amino acids in the sequence generated by the learning model with a predetermined probability, and selecting an amino acid after change using a probability density function such that the amino acid having a characteristic closer to that of the amino acid before change is selected with a higher probability;
- training the learning model to generate a sequence indicating a higher proxy evaluation value based on the proxy evaluation value for the sequence after the amino acid is changed, thereby generating a trained model; and
- generating a generation sequence using the trained model.
